# EUROPÄISCHE PATENTSCHRIFT

(11) **EP 3 268 349 B1**
(45) Veröffentlichungstag und Bekanntmachung des Hinweises auf die Patenterteilung: **08.01.2020**
(21) Anmeldenummer: 16709414.3
(22) Anmeldetag: 10.03.2016
(51) Int. Cl.: C01B 32/80, C07C 263/10, B01D 53/00, C01B 7/07, C07C 263/20

(54) **VERFAHREN ZUR TRENNUNG EINES PHOSGEN UND CHLORWASSERSTOFF ENTHALTENDEN STOFFSTROMS**
METHOD FOR THE SEPARATION OF A MATERIAL FLOW CONTAINING PHOSGENE AND HYDROGEN CHLORIDE
PROCÉDÉ DE SÉPARATION D'UN PHOSGÈNE ET FLUX DE MATIÈRE CONTENANT DU CHLORURE D'HYDROGÈNE

(30) Priorität: 12.03.2015 EP 15158862
(43) Veröffentlichungstag der Anmeldung: 17.01.2018
(73) Patentinhaber: BASF SE, 67056 Ludwigshafen (DE)
(72) Erfinder: SCHELLING, Heiner, 67273 Bobenheim am Berg (DE); MATTKE, Torsten, 67251 Freinsheim (DE); PALLASCH, Hans-Juergen, 67169 Kallstadt (DE); THIELE, Kai, 2040 Antwerpen 4 (BE)
(74) Vertreter: BASF IP Association
(86) Internationale Anmeldenummer: PCT/EP2016/055154
(87) Internationale Veröffentlichungsnummer: WO 2016/142475

(56) Entgegenhaltungen:
- EP-A1- 2 559 658
- WO-A1-2004/056758
- WO-A1-2006/029788
- US-A- 3 544 611

## Beschreibung

Die Erfindung betrifft ein Verfahren zur Trennung eines Phosgen und Chlorwasserstoff enthaltenden Stoffstroms, wobei der Chlorwasserstoff und Phosgen enthaltende Stoffstrom einer Destillationskolonne zugegeben wird, am Sumpf der Destillationskolonne ein Phosgen enthaltender Strom und am Kopf der Kolonne ein im Wesentlichen Chlorwasserstoff enthaltender Stoffstrom entnommen werden.

Phosgen und Chlorwasserstoff enthaltende Stoffströme fallen insbesondere bei der Aufarbeitung von Produktströmen der Isocyanatherstellung in einer Phosgenierung an.

Hierbei wird das korrespondierende Amin mit einem stöchiometrischen Überschuss an Phosgen kontaktiert und zu Isocyanat und Chlorwasserstoff abreagiert. Die Phosgenierung kann in den verschiedensten Varianten durchgeführt werden, zum Beispiel als Gasphasenphosgenierung, Kalt-Heißphosgenierung, einstufige flüssige Phosgenierung, als Hydrochloridphosgenierung und als Gas/Flüssigphosgenierung. Entsprechende Verfahren sind zum Beispiel in Ullmann's Encyclopedia of Industrial Chemistry, Chapter "Isocyanates, Organic", Chapter 4.1 und 4.2 (Six, C. and Richter, F. 2003. Isocyanates, Organic. Ullmann's Encyclopedia of Industrial Chemistry) beschrieben. Je nach Verfahrensvariante können Inerte oder Lösungsmittel genutzt werden. Das Reaktionsgemisch enthält im Wesentlichen das Isocyanat oder Vorprodukte desselben, zum Beispiel Carbamylchloride, den gebildeten Chlorwasserstoff, das Überschuss-Phosgen und gegebenenfalls die zugefügten Inerten und/oder Lösungsmittel.

Die Abtrennung des Chlorwasserstoffs aus dem Produktgemisch erfolgt im Wesentlichen, unabhängig von der Art der Aufarbeitung, gemeinsam mit dem Überschuss-Phosgen. Hinzu kommen teilweise Inerte und/oder Lösungsmittel.

Der Chlorwasserstoff als Reaktionsprodukt kann in verschiedenster Weise genutzt werden. So kann mittels oxidativer Dehydrierung, zum Beispiel mit Verfahren nach Deacon oder Kelchlor oder per Elektrolyse aus dem Chlorwasserstoff Chlor zur Synthese frischen Phosgens gewonnen werden. Des Weiteren kann der Chlorwasserstoff in Oxychlorierungen eingesetzt werden. Auch eine Absorption in Wasser zur Gewinnung von wässriger Salzsäure ist möglich. Dies ist zum Beispiel in Ullmann's Encyclopedia of Industrial Chemistry, "Isocyanates, Organic", Chapter 5 (Six, C. and Richter, F. 2003. Isocyanates, Organic. Ullmann's Encyclopedia of Industrial Chemistry), WO-A 97/243207, oder EP-A 0 876 335 beschrieben. In jedem Fall sollte zunächst der Chlorwasserstoff vom Überschuss-Phosgen getrennt werden. Auf Grund der teilweise erheblichen Überschüsse gebietet schon die Wirtschaftlichkeit der Verfahren eine Rückgewinnung des Phosgens und dessen Rückführung in den Prozess. Eine Trennung von Chlorwasserstoff und Phosgen ist daher wesentlicher Bestandteil von Isocyanat-Verfahren auf Basis der Phosgenierung. Für die vorstehend beschriebene Trennung können verschiedenste thermische Trennmethoden Anwendung finden. Am weitesten verbreitet sind Absorption und Destillation. Andere Methoden wie Membranverfahren sind zum Beispiel aus WO-A 2013/026591 bekannt.

Bei der Absorption wird Phosgen durch ein Absorptionsmittel aus einem gasförmigen, Chlorwasserstoff und Phosgen enthaltenden Feedstrom ausgewaschen. Bei Verfahren mit Lösungsmittel wird dieses häufig als Absorptionsmittel genutzt. Solche Verfahren sind zum Beispiel in EP- A 1 849 767 beschrieben. Nachteil dieses Verfahrens ist jedoch zum einen, dass in dem Chlorwasserstoff Spuren des häufig organischen Absorptionsmittels enthalten sind und diese zu Problemen in der Verwendung des Chlorwasserstoffs beispielsweise im Deacon-Prozess oder bei der Elektrolyse führen können. Gegebenenfalls muss durch weitere thermische Trennstufen wie Adsorption, zum Beispiel wie in EP-A 1 981 619 oder WO-A 2008/122363 beschrieben, oder Destillation/Rektifikation, wie beispielsweise in der US 6,719,957 oder EP-A 2 021 275 beschrieben, eine Abtrennung des Absorptionsmittels aus dem Chlorwasserstoff erfolgen. Ein weiterer Nachteil der Absorption ist der hohe Anteil von Absorptionsmittel im ausgewaschenen Phosgen sowie Anteile gelösten Chlorwasserstoffs, die ebenfalls mit ausgewaschen werden und bei Rückführung des Stroms in die Reaktion nachteilig sein können, wie dies zum Beispiel aus DE-A 10 2004 026 095 bekannt ist. Daher kann eine weitere Trennstufe vor oder nach der Absorption notwendig werden um den Chlorwasserstoffgehalt im zurückgeführten Phosgen sowie den Absorptionsmittelanteil zu minimieren.

Die vorstehend beschriebenen Nachteile der Absorption können durch eine destillative Trennung von Chlorwasserstoff und Phosgen vermieden werden. Der große Siedepunktunterschied zwischen Chlorwasserstoff und Phosgen lässt eine destillative Trennung zu, erfordert jedoch durch den hohen Dampfdruck des Chlorwasserstoffs eine Trennung bei tiefen Kopftemperaturen oder einen entsprechend hohen Druck.

Tiefe Temperaturen bedingen hohe Investitions- und Betriebskosten für eine entsprechende Kälteanlage. Hoher Druck erfordert in der Regel eine Verdichtung der Reaktionsgase oder zumindest von Teilen davon beziehungsweise einen entsprechend höheren Druck in der Reaktion. Letzterer kann nachteilig für Ausbeute und Qualität sein. Die Verdichtung von Chlorwasserstoff/Phosgen-Gasgemischen stellt hohe Anforderungen an Sicherheit, Investition und Betriebskosten.

Es war Aufgabe der Erfindung die oben beschriebenen Nachteile der aus dem Stand der Technik beschriebenen Verfahren zur Trennung von Chlorwasserstoff/Phosgen-Gemischen zu vermeiden.

Gelöst wird die Aufgabe durch ein Verfahren zur Trennung eines Phosgen und Chlorwasserstoff enthaltenden Stoffstroms, wobei der Chlorwasserstoff und Phosgen enthaltende Stoffstrom einer Destillationskolonne zugegeben wird, am Sumpf der Destillationskolonne ein Phosgen enthaltender Strom und am Kopf der Destillationskolonne ein im Wesentlichen Chlorwasserstoff enthaltender Stoffstrom entnommen werden, und zumindest ein Teil des am Kopf entnommenen Stoffstroms komprimiert und zumindest teilweise kondensiert wird und mindestens ein Teil des flüssigen und komprimierten im Wesentlichen Chlorwasserstoff enthaltenden Stoffstroms entspannt und am Kopf in die Destillationskolonne als Rücklauf zurückgeführt wird.

Durch die Kompression zumindest eines Teils des am Kopf der Kolonne entnommenen im Wesentlichen Chlorwasserstoff enthaltenden Stoffstroms ist es möglich, die Kondensation bei höheren Temperaturen und damit mit einem deutlich reduzierten Energieverbrauch durchzuführen. Üblicherweise wird eine Kompression möglichst vermieden, da diese mit einem hohen Energiebedarf verbunden ist. Zudem wird üblicherweise angestrebt, Phosgen bei moderaten Drücken handzuhaben. Aus diesem Grund wird üblicherweise auf eine Verdichtung des Rohgasstroms aus Phosgen und Chlorwasserstoff verzichtet und eine absorptive Trennung unter Einsatz eines Lösungsmittels angewandt. Als Betriebsdruck wird bei herkömmlichen Verfahren hierzu der Druck gewählt, bei dem das gasförmige Gemisch im Verfahren anfällt.

Der der Destillationskolonne zugeführte Stoffstrom entstammt zum Beispiel einer Herstellung von Isocyanaten aus dem korrespondierenden Amin und Phosgen. Die Herstellung des Isocyanats kann dabei sowohl in der Gasphase als auch in der Flüssigphase durchgeführt werden. Bei der Gasphasenphosgenierung enthält der Stoffstrom in der Regel Chlorwasserstoff und Phosgen sowie gegebenenfalls leicht siedende Nebenkomponenten oder Verunreinigungen. Bei der Flüssigphasenphosgenierung kann zusätzlich Lösungsmittel enthalten sein.

Die Destillation des Phosgen und Chlorwasserstoff enthaltenden Stoffstroms erfolgt vorzugsweise bei dem Druck, bei dem der Stoffstrom der Isocyanatherstellung entnommen worden ist. Geeignete Drücke liegen dabei im Allgemeinen in einem Bereich von 1 bis 10 bar (abs), bevorzugt im Bereich von 1,2 bis 8 bar (abs) und insbesondere im Bereich von 1,5 bis 7 bar (abs).

Entsprechend liegt der Druck des am Kopf der Destillationskolonne entnommenen Stoffstroms bei dem Druck, bei dem auch die Destillationskolonne betrieben wird. Komprimiert wird der am Kopf der Destillationskolonne entnommene Stoffstrom vorzugsweise auf einen Druck im Bereich von 5 bis 25 bar, mehr bevorzugt auf einen Druck im Bereich von 7 bis 22 bar und insbesondere auf einen Druck im Bereich von 10 bis 20 bar.

In einer Ausführungsform der Erfindung umfasst die Destillationskolonne einen Verstärkungsteil und einen Abtriebsteil und der Phosgen und Chlorwasserstoff enthaltende Stoffstrom wird als Seitenzulauf zwischen Verstärkungsteil und Abtriebsteil zugeführt. Durch die Verwendung einer Destillationskolonne mit Verstärkungsteil und Abtriebsteil wird eine verbesserte Aufreinigung des am Sumpf anfallenden Phosgens erzielt. Insbesondere kann hierdurch erreicht werden, dass das am Sumpf anfallende Phosgen im Wesentlichen frei von Chlorwasserstoff ist. Im Wesentlichen frei bedeutet in diesem Zusammenhang, dass der Anteil an Chlorwasserstoff im Bereich von 10 bis 1000 ppm liegt. Dies erlaubt es, das Phosgen ohne weitere Aufarbeitung direkt in die Isocyanatherstellung zurückzuführen, wobei durch die Abwesenheit des Chlorwasserstoffs die Bildung von Aminhydrochloriden als unerwünschte Nebenprodukte nicht bereits bei der Mischung des Amins mit dem Phosgen beginnt sondern erst verzögert, was weiterhin den Vorteil mit sich bringt, dass die Menge an gebildetem Aminhydrochlorid geringer gehalten wird als bei der Zugabe von Phosgen, das noch Reste an Chlorwasserstoff enthält.

Wenn keine Destillationskolonne mit Verstärkungsteil und Abtriebsteil eingesetzt wird, sondern eine nur einen Verstärkungsteil aufweisende Destillationskolonne, erfolgt die Zufuhr des Phosgen und Chlorwasserstoff enthaltenden Stoffstroms am Sumpf der Destillationskolonne.

Der Verstärkungsteil und, sofern vorhanden, der Abtriebsteil enthalten unabhängig voneinander vorzugsweise Einbauten, zum Beispiel in Form strukturierter Packungen, Füllkörperschüttungen oder in Form von Böden, wie sie in Destillationskolonnen eingesetzt werden können. Bevorzugt ist jedoch der Einsatz einer oder mehrerer Packungen als Einbauten im Verstärkungsteil und im Abtriebsteil.

Um die Aufreinigung des am Kopf der Destillationskolonne entnommenen und im Wesentlichen Chlorwasserstoff enthaltenden Stoffstroms zu verbessern, ist es vorteilhaft, den am Kopf entnommenen und im Wesentlichen Chlorwasserstoff enthaltenden Stoffstrom nach dem Komprimieren einer Verstärkerkolonne zuzuführen und den am Sumpf der Verstärkerkolonne anfallende Strom am Kopf in die Destillationskolonne zurückzuführen. Der am Kopf der Verstärkerkolonne anfallende Stoffstrom wird vorzugsweise in einem Kondensator teilkondensiert, der kondensierte Teil in die Verstärkerkolonne zurückgeführt und der nicht kondensierte, gasförmige, aufgereinigte Chlorwasserstoff enthaltende Anteil entnommen und einer Weiterverwertung, beispielsweise einer Salzsäureproduktion oder einer Chlorherstellung durch Oxidation des Chlorwasserstoffs, zugeführt.

Ist der Verstärkerteil der Destillationskolonne ausreichend dimensioniert, dann kann auf die Verstärkerkolonne auf der Druckseite des Kompressors verzichtet werden. Es ist dann nur noch die Kondensation des Anteils an Chlorwasserstoff erforderlich, der für den flüssigen Rücklauf in die auf niedrigerem Druck betriebene Destillationskolonne erforderlich ist.

Zur Energieeinsparung ist es bevorzugt, Wärmeintegrationsmaßnahmen einzusetzen. Für die Wärmeintegration bietet sich insbesondere der kalte, nicht kondensierte Gasstrom aus dem Kopfkondensator an. Dieser kann für die Vorkühlung anderer Ströme genutzt werden. Auch der kalte, am Kopf der Kolonne anfallende Stoffstrom ist für energieintegrative Maßnahmen nutzbar. Auch sind Zwischenkühlungen zur Minimierung der Energiekosten denkbar. Die Möglichkeiten zur Energieintegration und mithin zur Minimierung des Energieverbrauchs sind sehr vielfältig und dem Fachmann bekannt.

In einer Ausführungsform gibt der kondensierte und im Wesentlichen Chlorwasserstoff enthaltende Stoffstrom in einem Wärmeübertrager Wärme an den zu kondensierenden, Chlorwasserstoff enthaltenden Stoffstrom ab, bevor dieser komprimiert wird. Nach dem Komprimieren wird der Chlorwasserstoff enthaltende Stoffstrom abgekühlt und teilkondensiert und der nicht kondensierte Teil wird über einen Gasabzug entnommen. Der kondensierte Teil wird in den Wärmeübertrager als Temperiermedium zum Erwärmen des am Kopf entnommenen und im Wesentlichen Chlorwasserstoff enthaltenden Stoffstroms zurückgeführt.

In einer weiteren Ausführungsform nimmt der nicht kondensierte Teil des im Wesentlichen Chlorwasserstoff enthaltenden Stoffstroms in einem Wärmetauscher von dem komprimierten zu kondensierenden Chlorwasserstoff enthaltenden Stoffstrom Wärme auf. Hierdurch wird der zu kondensierende Chlorwasserstoff enthaltende Stoffstrom vorgekühlt, bevor dieser dem Kondensator zugeführt wird.

Weiterhin ist es möglich, der Destillationskolonne einen Gasstrom über einen Seitenabzug zu entnehmen, diesen in einem ersten Kühler zumindest teilweise zu kondensieren, den flüssigen Anteil in die Destillationskolonne zurückzuführen und den gasförmigen Teil einem zweiten Kühler zuzuführen, wobei der Gasstrom im zweiten Kühler Wärme an den am Kopf entnommenen und im Wesentlichen Chlorwasserstoff enthaltenden Stoffstrom abgibt. Hierbei ist es bevorzugt, wenn der am Kopf entnommene Stoffstrom vor Zufuhr in den zweiten Kühler in einem Wärmeübertrager aufgeheizt wird. Um Energie effektiv zu nutzen, ist es dabei bevorzugt, wenn der am Kopf entnommene Stoffstrom in dem Wärmeübertrager Wärme von dem am Kopf in die Destillationskolonne zurückgeführten kondensierten Stoffstrom aufnimmt.

Die einzelnen vorstehend beschriebenen Wärmeintegrationsmaßnahmen können jeweils einzeln oder auch in beliebigen Kombinationen umgesetzt werden.

Einige Beispiele für mögliche Ausführungsformen sind in den Figuren dargestellt und werden in der nachfolgenden Beschreibung näher erläutert.

Es zeigen:
- Figur 1: das erfindungsgemäße Verfahren in einer ersten Ausführungsform,
- Figur 2: das erfindungsgemäße Verfahren in einer zweiten Ausführungsform,
- Figur 3: das erfindungsgemäße Verfahren mit Vorkühlung/Teilkondensation des zuzuführenden Phosgens und Chlorwasserstoff enthaltenden Stoffstroms,
- Figur 4: das erfindungsgemäße Verfahren in einer Destillationskolonne mit Verstärkungsteil und Abtriebsteil,
- Figur 5: das erfindungsgemäße Verfahren mit zusätzlicher Verstärkungskolonne,
- Figur 6: das erfindungsgemäße Verfahren mit zusätzlichen Wärmeintegrationsmaßnahmen.

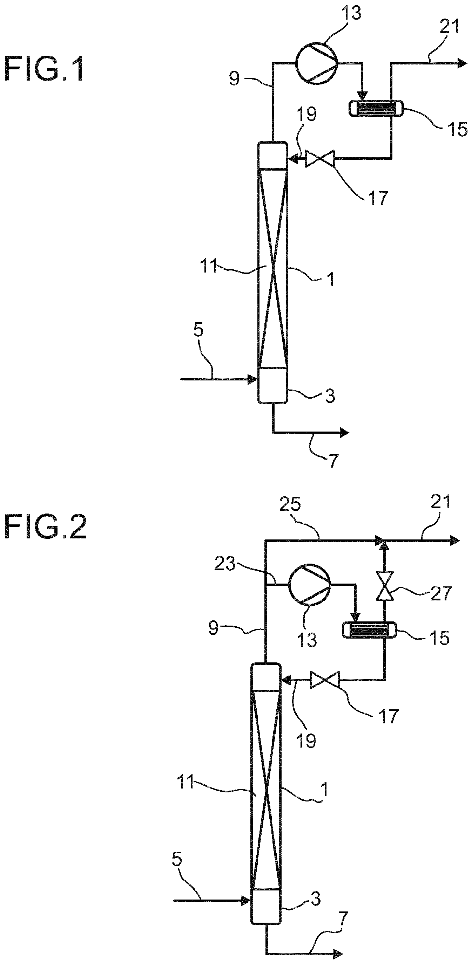

Figur 1 zeigt das erfindungsgemäße Verfahren in einer ersten Ausführungsform.

Einer Destillationskolonne 1 wird im unteren Bereich 3 über einen Zulauf 5 ein Phosgen und Chlorwasserstoff enthaltender, gasförmiger Stoffstrom zugeführt. In der Destillationskolonne wird der Phosgen und Chlorwasserstoff enthaltende Stoffstrom destillativ in einen Phosgen enthaltenden Sumpfstrom, der über einen Sumpfabzug 7 entnommen wird, und einen im Wesentlichen Chlorwasserstoff enthaltenden Stoffstrom, der an einem Kopfabzug 9 entnommen wird, getrennt.

Der Phosgen und Chlorwasserstoff enthaltende Stoffstrom entstammt vorzugsweise einer Isocyanatherstellung. Der Druck bei dem die Destillation durchgeführt wird, entspricht dabei in einer Ausführungsform dem Druck, mit dem der Phosgen und Chlorwasserstoff enthaltende Stoffstrom der Isocyanatherstellung entnommen wurde. In einer alternativen Ausführungsform wird die Destillation bei einem Druck durchgeführt, der kleiner ist als der Druck, mit dem der Phosgen und Chlorwasserstoff enthaltende Stoffstrom der Isocyanatherstellung entnommen wurde.

Zur Unterstützung der Destillation weist die Destillationskolonne 1 Einbauten 11 auf, beispielsweise eine strukturierte Packung oder eine Füllkörperschüttung. Alternativ ist es auch möglich, eine Bodenkolonne als Destillationskolonne 1 einzusetzen.

Erfindungsgemäß wird der über den Kopfabzug 9 entnommene und im Wesentlichen Chlorwasserstoff enthaltende Stoffstrom in einem Kompressor 13 auf einen höheren Druck verdichtet. Der Druck, auf den der im Wesentlichen Chlorwasserstoff enthaltende Stoffstrom verdichtet wird, liegt vorzugsweise im Bereich von 5 bis 25 bar.

Nach der Kompression wird der im Wesentlichen Chlorwasserstoff enthaltende Stoffstrom in einen Kopfkondensator 15 geleitet. Im Kopfkondensator erfolgt eine Teilkondensation des im Wesentlichen Chlorwasserstoff enthaltenden Stoffstroms. Aufgrund des hohen Drucks ist es hierbei nicht erforderlich, auf die Temperaturen abzukühlen, die erforderlich wären, wenn die Kondensation bei dem am Kopf der Destillationskolonne herrschenden Druck durchgeführt worden wäre. Der kondensierte Teil des im Wesentlichen Chlorwasserstoff enthaltenden Stoffstroms wird in einem Entspannungsorgan 17, beispielsweise einer Drossel oder einem Ventil auf den am Kopf der Destillationskolonne 1 herrschenden Druck entspannt. Dabei kühlt sich dieser Stoffstrom unter teilweiser Verdampfung auf die Siedetemperatur bei Kolonnendruck ab und wird über einen Rücklauf 19 am Kopf in die Destillationskolonne 1 zurückgeführt. Der nicht kondensierte Teil des im Wesentlichen Chlorwasserstoff enthaltenden Stoffstroms wird über einen Gasabzug 21 aus dem Kopfkondensator 15 entnommen.

In Figur 2 ist das erfindungsgemäße Verfahren in einer zweiten Ausführungsform dargestellt.

Im Unterschied zu der in Figur 1 dargestellten Ausführungsform wird bei der in Figur 2 dargestellten Ausführungsform der über den Kopfabzug 9 entnommene, im Wesentlichen Chlorwasserstoff enthaltende Stoffstrom in einen ersten Teilstrom 23 und einen zweiten Teilstrom 25 geteilt. Der erste Teilstrom 23 wird dem Kompressor 13 zugeführt, in diesem auf den höheren Druck komprimiert und im Kopfkondensator 15 teilweise kondensiert. Der kondensierte Teil des im Wesentlichen Chlorwasserstoff enthaltenden Stoffstroms wird auch hier in einem Entspannungsorgan 17 entspannt und über den Rücklauf 19 in die Destillationskolonne 1 zurückgeführt. Der gasförmige Anteil wird in einem zweiten Entspannungsorgan 27 entspannt und aus dem Prozess entnommen. Hierzu ist es - wie in Figur 2 dargestellt - besonders bevorzugt, wenn der entspannte gasförmige Anteil in den zweiten Teilstrom 25 eingeleitet wird und sowohl der gasförmige Anteil als auch der zweite Teilstrom 25 über einen gemeinsamen Gasabzug 21 aus dem Prozess abgeführt werden.

Der erste Teilstrom 23 enthält vorzugsweise 5 bis 50 Vol.-%, insbesondere 10 bis 40 Vol.-% des über den Kopfabzug 9 entnommenen und im Wesentlichen Chlorwasserstoff enthaltenden Stoffstroms.

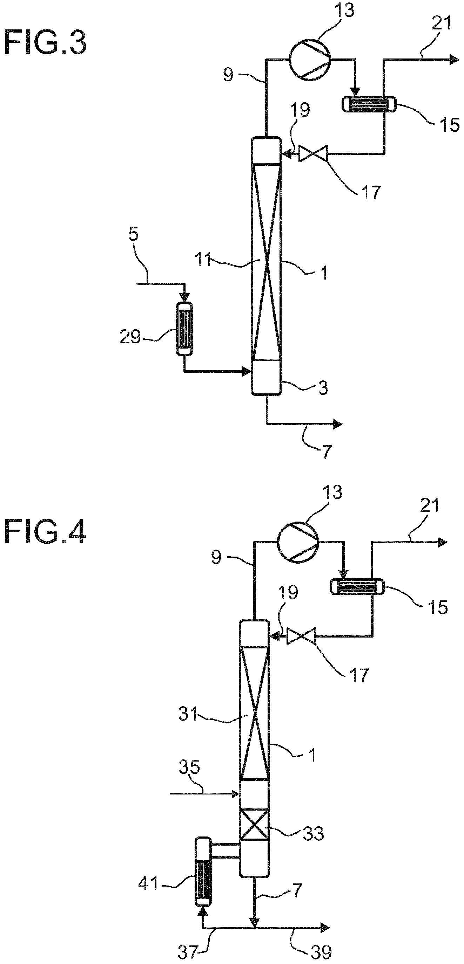

Eine weitere Ausführungsform des erfindungsgemäßen Verfahrens ist in Figur 3 dargestellt. Im Unterschied zu der in Figur 1 dargestellten Ausführungsform wird bei der in Figur 3 dargestellten Ausführungsform der zuzuführende Phosgen und Chlorwasserstoff enthaltende Stoffstrom ein- oder mehrstufig vorgekühlt und/oder teilkondensiert.

Hierzu wird der Phosgen und Chlorwasserstoff enthaltende Stoffstrom einem oder mehreren Wärmetauschern 29 zugeführt, in dem/denen dieser vorgekühlt und/oder teilkondensiert wird. Die Wärmetauscher 29 sind dabei vorzugsweise indirekte Wärmetauscher, in dem/denen Wärme an einen geeigneten Wärmeträger von dem Phosgen und Chlorwasserstoff enthaltenden Stoffstrom abgegeben wird. Üblicherweise eingesetzte Wärmeträger sind zum Beispiel Thermalöle. Auch eine Wärmeintegration mit anderen Prozessströmen ist möglich.

Auch bei der in Figur 3 dargestellten Ausführungsform ist es alternativ möglich, wie bei der in Figur 2 dargestellten Ausführungsform, nicht den gesamten über den Kopfabzug 9 entnommenen im Wesentlichen Chlorwasserstoff enthaltenden Stoffstrom sondern nur einen Teil des über den Kopfabzug 9 entnommenen im Wesentlichen Chlorwasserstoff enthaltenden Stoffstrom dem Kopfkondensator 15 zuzuführen.

Um am Sumpf einen Stoffstrom zu erhalten, der im Wesentlichen frei von Chlorwasserstoff ist, ist es möglich, die Destillationskolonne 1 nicht als reine Verstärkungskolonne wie in den in den Figuren 1 bis 3 dargestellten Ausführungsformen zu betreiben sondern als Destillationskolonne 1 mit Verstärkungsteil 31 und Abtriebsteil 33. Eine entsprechende Ausführungsform ist in Figur 4 dargestellt. Wenn die Destillationskolonne 1 einen Verstärkungsteil 31 und einen Abtriebsteil 33 umfasst, ist der Zulauf 5 für den Phosgen und Chlorwasserstoff enthaltenden Stoffstrom nicht im unteren Bereich 3 der Destillationskolonne angeordnet sondern als Seitenzulauf 35 zwischen dem Verstärkungsteil 31 und dem Abtriebsteil 33.

Zusätzlich ist es in diesem Fall vorteilhaft, wenn der über den Sumpfabzug 7 entnommene Stoffstrom in einen ersten Sumpfteilstrom 37 und einen zweiten Sumpfteilstrom 39 geteilt wird. Der Anteil des ersten Sumpfteilstroms 37 bezogen auf den gesamten über den Sumpfabzug 7 entnommenen hängt dabei vom Rücklauf und der verwendeten Verdampferart ab. Der erste Sumpfteilstrom 37 wird einem Verdampfer 41 zugeführt, in dem dieser zumindest teilweise verdampft und anschließend erneut der Destillationskolonne 1 im unteren Bereich 3 wieder zugeführt wird.

Auch hier ist es möglich, den zuzuführenden Phosgen und Chlorwasserstoff enthaltenden Stoffstrom wie in der in Figur 3 dargestellten Ausführungsform vorzukühlen und/oder teilzukondensieren und/oder wie in der in Figur 2 dargestellten Ausführungsform nur einen Teilstrom des über den Kopfabzug 9 entnommenen und im Wesentlichen Chlorwasserstoff enthaltenden Stoffstrom anstelle des gesamten über den Kopfabzug 9 entnommenen im Wesentlichen Chlorwasserstoff enthaltenden Stoffstroms in den Kompressor und anschließend in den Kopfkondensator einzuleiten.

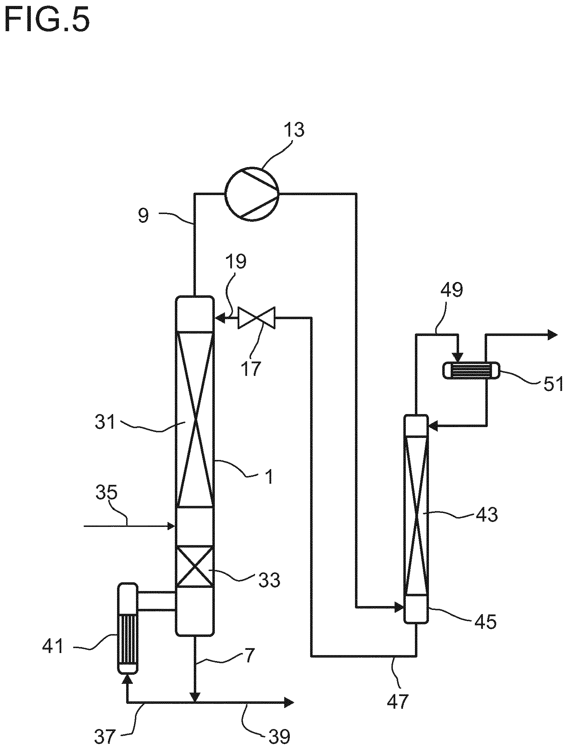

Das in Figur 5 dargestellte Verfahren unterscheidet sich von dem in Figur 4 durch eine zusätzliche Verstärkungskolonne 43. Durch die zusätzliche Verstärkerkolonne 43 ist es möglich, den im Wesentlichen Chlorwasserstoff enthaltenden Stoffstrom noch weiter aufzukonzentrieren und noch im über den Kopfabzug 9 entnommenen im Wesentlichen Chlorwasserstoff enthaltenden Stoffstrom enthaltene Verunreinigungen, beispielsweise Phosgen, zu entfernen.

Die Verstärkungskolonne 43 ist stromab des Kompressors 13 geschaltet, so dass der im Wesentlichen Chlorwasserstoff enthaltende Stoffstrom der Verstärkungskolonne 43 nach der Kompression im Kompressor 13 in die Verstärkungskolonne 43 eingeleitet wird. Der Zulauf für den komprimierten im Wesentlichen Chlorwasserstoff enthaltenden Stoffstrom liegt dabei im unteren Bereich 45 der Verstärkungskolonne 43, vorzugsweise unterhalb in der Verstärkungskolonne 43 enthaltenen Einbauten, beispielsweise Böden, einer strukturierten Packung oder einer Füllkörperschüttung. In der Verstärkungskolonne 43 fällt ein Sumpfstrom an, der über einen Sumpfabzug 47 entnommen und im Entspannungsorgan 19 entspannt wird. Anschließend wird der am Sumpfabzug 47 der Verstärkungskolonne 43 anfallende Sumpfstrom wieder am Kopf in die Destillationskolonne 1 zurückgeführt.

Der am Kopf der Verstärkungskolonne 43 anfallende Stoffstrom wird über einen Kopfabzug 49 entnommen und in einen Kopfkondensator 51 eingeleitet. Im Kopfkondensator 51 erfolgt eine Teilkondensation des am Kopf der Verstärkungskolonne 43 anfallenden Stoffstroms. Der kondensierte, flüssige Teil wird am Kopf zurück in die Verstärkungskolonne 43 zurückgeführt und der nicht kondensierte, gasförmige Teil wird abgeführt und beispielsweise einer Salzsäureherstellung oder der Oxidation von Chlorwasserstoff zur Chlorherstellung zugeführt.

Auch bei der in Figur 5 dargestellten Ausführungsform ist es möglich, den zugeführten Phosgen und Chlorwasserstoff enthaltenden Stoffstrom vor Zugabe in die Destillationskolonne vorzukühlen und/oder teilzukondensieren und/oder nur einen Teilstrom dem Kompressor zuzuführen. In diesem Fall ist es besonders vorteilhaft, wenn der gesamte am Kopf der Destillationskolonne 1 entnommene Stoffstrom dem Kompressor 13 und anschließend der Verstärkungskolonne 43 zugeführt wird.

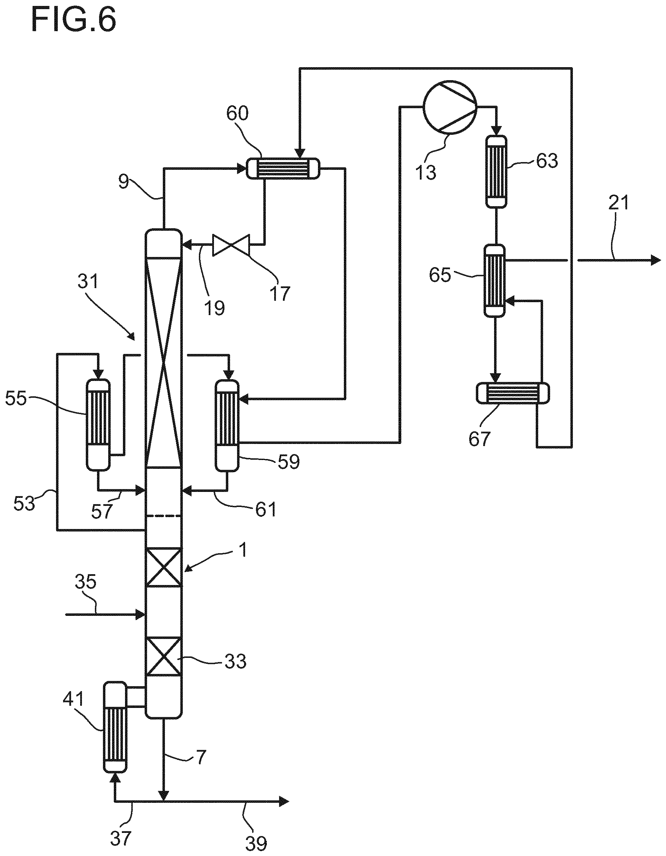

Figur 6 zeigt das erfindungsgemäße Verfahren mit zusätzlichen Wärmeintegrationsmaßnahmen.

Für eine erste Wärmeintegrationsmaßnahme wird der Destillationskolonne 1 im Verstärkungsteil 31 ein Gasstrom über einen Seitenabzug 53 entnommen. Um den Gasstrom entnehmen zu können, weist der Verstärkungsteil 31 in der hier dargestellten Ausführungsform zwei getrennte Einbauten auf. der Seitenabzug 53 ist dabei zwischen den getrennten Einbauten des Verstärkungsteils 31 angeordnet.

Der Gasstrom wird nach der Entnahme über den Seitenabzug 53 einem ersten Kondensator 55 zugeführt. Im ersten Kondensator 55 wird der Gasstrom teilweise kondensiert. Der flüssige Teil wird über einen ersten Seitenzulauf 57 vorzugsweise zwischen den Einbauten des Verstärkungsteils zugeführt. Der nicht kondensierte Teil wird einem zweiten Kondensator 59 zugeführt. Erfindungsgemäß wird der erste Kondensator 55 mit einer externen Energieversorgung betrieben. In dieser Ausführungsform wird der Kopfstrom 9 zunächst einem Wärmeübertrager 60 zugeführt. Der den Wärmeübertrager 60 verlassende im Wesentlichen Chlorwasserstoff enthaltende Strom wird im zweiten Kondensator 59 genutzt, um den gasförmigen Teil weiter zu kondensieren. Der auf diese Weise teilkondensierte Strom wird über einen zweiten Seitenzulauf 61 vorzugsweise auf der gleichen Höhe wie der erste Seitenzulauf 57 in die Destillationskolonne 1 zurückgeführt. Der erste Seitenzulauf 57 und der zweite Seitenzulauf 61 können allerdings auch auf unterschiedlichen Höhen angeordnet sein. Bevorzugt ist es aber auch in diesem Fall, dass der erste Seitenzulauf 57 und der zweite Seitenzulauf 61 oberhalb des Seitenabzugs 53 und zwischen den Einbauten des Verstärkungsteils 31 angeordnet sind.

Durch die Wärmeabgabe von dem teilweise zu kondensierenden Strom nimmt der im Wesentlichen Chlorwasserstoff enthaltende Stoffstrom vom Kopf der Kolonne Wärme im zweiten Kondensator 59 auf. Nach Verlassen des zweiten Kondensators 59 wird der im Wesentlichen Chlorwasserstoff enthaltende Stoffstrom dem Kompressor 13 zugeführt und auf einen höheren Druck komprimiert. Der komprimierte Stoffstrom wird in einem ersten Kühler 63 abgekühlt, dann einem zweiten Kühler 65 und abschließend einem Kondensator 67 zugeführt. Im Kondensator 67 erfolgt eine Teilkondensation des im Wesentlichen Chlorwasserstoff enthaltenden Stoffstroms. Der flüssige Teil wird dem Wärmeübertrager 60 als Temperiermedium zugeführt. Im Wärmeübertrager 60 gibt der flüssige Teil Wärme an den über den Kopfabzug 9 entnommenen im Wesentlichen Chlorwasserstoff enthaltenden Stoffstrom ab und erwärmt diesen dadurch. Der erwärmte im Wesentlichen Chlorwasserstoff enthaltende Stoffstrom wird dann - wie vorstehend beschrieben - dem zweiten Kondensator 59 zugeführt. Der im Wärmeübertrager 60 weiter abgekühlte flüssige Teil wird im Entspannungsorgan 17 entspannt und am Kopf zurück in die Destillationskolonne 1 geleitet.

Der aus dem Kondensator 67 entnommene gasförmige Teil wird in den zweiten Kühler 65 als Kältemittel eingeleitet, wodurch der gasförmige Teil Wärme von dem zu kondensierenden, komprimierten und im Wesentlichen Chlorwasserstoff enthaltenden Stoffstrom aufnimmt, bevor dieser in den Kondensator 67 eingeleitet wird. Der im zweiten Kühler erwärmte Teil wird über den Gasabzug 21 aus dem Prozess entnommen.

Neben der in Figur 6 dargestellten Kopplung der Wärmeintegrationsmaßnahmen können diese auch in jeder beliebigen anderen Kombination oder auch nur einzeln eingesetzt werden. Zudem ist es auch noch möglich, den Phosgen und Chlorwasserstoff enthaltenden Stoffstrom vor der Zugabe in die Destillationskolonne 1 - wie in Figur 3 dargestellt - vorzukühlen beziehungsweise teilzukondensieren.

Gegebenenfalls kann es in einigen Varianten vorteilhaft sein die Verdichtung mehrstufig, dann gegebenenfalls mit Zwischenkühlung auszuführen.

### Beispiele

### Vergleichsbeispiel

Einer Absorptionskolonne werden am Sumpf 35000 kg/h eines 28,6 Gew.-% Chlorwasserstoff, 62,9 Gew.-% Phosgen und 8,6 Gew.-% Chlorbenzol enthaltenden Stoffstroms mit einer Temperatur von 90°C und am Kopf 11254 kg/h Chlorbenzol mit einer Temperatur von -25°C zugeführt. Der Chlorwasserstoff, Phosgen und Chlorbenzol enthaltende Stoffstrom und das Chlorbenzol werden im Gegenstrom geführt. Am Sumpf der Absorptionskolonne fallen 37670 kg/h eines 3,8 Gew.-% Chlorwasserstoff (1431 kg/h), 58,4 Gew.-% Phosgen und 37,8 Gew.-% Chlorbenzol enthaltenden Stoffstroms mit einer Temperatur von -7,6°C an. Der am Kopf der Destillationskolonne anfallende Stoffstrom wird in einem Kondensator teilkondensiert, der kondensierte Teil wird in die Absorptionskolonne zurückgeführt und der gasförmige Anteil wird aus dem Prozess entnommen. Der Massenstrom des gasförmigen Anteils beträgt 8584 kg/h und er enthält 99,8 Gew.-% Chlorwasserstoff und jeweils 0,1 Gew.-% Phosgen und Chlorbenzol und weist eine Temperatur von -25°C auf. Die Absorption wird bei einem Druck von 2,2 bar durchgeführt.

### Beispiel

Anstelle einer Absorptionskolonne wie im Vergleichsbeispiel wird eine Destillationskolonne entsprechend dem in Figur 3 dargestellten Aufbau zur Aufarbeitung des Chlorwasserstoff, Phosgen und Chlorbenzol enthaltenden Stoffstroms eingesetzt. Die Zusammensetzung des der Destillationskolonne zugeführten Stoffstroms entspricht dabei der des Vergleichsbeispiels.

Bei einem Massenstrom von 35000 kg/h des Chlorwasserstoff, Phosgen und Chlorbenzol enthaltenden Stoffstroms mit einer Temperatur von 90°C fallen am Sumpf der Destillationskolonne 26212 kg/h eines 4,7 Gew.-% Chlorwasserstoff (1232 kg/h), 83,9 Gew.-% Phosgen und 11,4 Gew.-% Chlorbenzol enthaltenden Stoffstroms mit einer Temperatur von -15.4°C an. Die Destillation wird dabei bei einem Druck von 2,2 bar durchgeführt.

Der am Kopf anfallende Gasstrom wird auf einen Druck von 13,2 bar komprimiert und dem Kopfkondensator zugeführt. Der kondensierte flüssige Teilstrom wird expandiert und in die Destillationskolonne zurückgeführt. Der Gasstrom wird mit einer Temperatur von -22,8°C entnommen. Der dem Kopfkondensator entnommene Gasstrom beträgt 8788 kg/h und enthält 99,9 Gew.-% Chlorwasserstoff und 0,1 Gew.-% Phosgen.

Im Unterschied zur Absorption enthält der am Sumpf anfallende, Phosgen enthaltende, Stoffstrom sehr viel weniger Lösungsmittel und auch weniger Chlorwasserstoff. Eine weitere Aufarbeitung dieses Stroms vor erneuter Nutzung in der Reaktion kann entweder entfallen oder aber deutlich vereinfacht werden. Der Gasstrom hinter dem Kopfkondensator ist frei von Lösungsmittel, so dass dieser ohne weitere Aufarbeitung zum Beispiel in eine oxidative Dehydrierung (Deacon-Verfahren) oder eine HCl-Elektrolyse geführt werden kann.

### Bezugszeichenliste

- 1: Destillationskolonne
- 3: unterer Bereich der Destillationskolonne 1
- 5: Zulauf
- 7: Sumpfabzug
- 9: Kopfabzug
- 11: Einbauten
- 13: Kompressor
- 15: Kopfkondensator
- 17: Entspannungsorgan
- 19: Rücklauf zum Kopf der Kolonne
- 21: Gasabzug
- 23: erster Teilstrom
- 25: zweiter Teilstrom
- 27: zweites Entspannungsorgan
- 29: Wärmetauscher
- 31: Verstärkungsteil
- 33: Abtriebsteil
- 35: Seitenzulauf
- 37: erster Sumpfteilstrom
- 39: zweiter Sumpfteilstrom
- 41: Verdampfer
- 43: Verstärkungskolonne
- 45: unterer Bereich der Verstärkungskolonne 43
- 47: Sumpfabzug der Verstärkungskolonne 43
- 49: Kopfabzug der Verstärkungskolonne 43
- 51: Kopfkondensator der Verstärkungskolonne 43
- 53: Seitenabzug
- 55: erster Kondensator
- 57: erster Seitenzulauf
- 59: zweiter Kondensator
- 60: Wärmeübertrager
- 61: zweiter Seitenzulauf
- 63: erster Kühler
- 65: zweiter Kühler
- 67: Kondensator

## Patentansprüche

1. Verfahren zur Trennung eines Phosgen und Chlorwasserstoff enthaltenden Stoffstroms (5; 35), wobei der Chlorwasserstoff und Phosgen enthaltende Stoffstrom (5; 35) einer Destillationskolonne (1) zugegeben wird, am Sumpf der Destillationskolonne (1) ein Phosgen enthaltender Strom (7) und am Kopf der Destillationskolonne (1) ein Chlorwasserstoff enthaltender Stoffstrom (9) entnommen werden, **dadurch gekennzeichnet, dass** zumindest ein Teil des am Kopf entnommenen Stoffstroms (9) komprimiert und zumindest teilweise kondensiert wird und mindestens ein Teil des flüssigen und komprimierten Chlorwasserstoff enthaltenden Stoffstroms entspannt und am Kopf in die Destillationskolonne (1) als Rücklauf zurückgeführt wird.

2. Verfahren gemäß Anspruch 1, **dadurch gekennzeichnet, dass** der Stoffstrom (9) am Kopf bei einem Druck im Bereich von 1 bis 10 bar entnommen und vor dem Kondensieren auf einen Druck im Bereich von 5 bis 25 bar komprimiert wird.

3. Verfahren gemäß Anspruch 1 oder 2, **dadurch gekennzeichnet, dass** die Destillationskolonne (1) einen Verstärkungsteil (31) und einen Abtriebsteil (33) umfasst und der Phosgen und Chlorwasserstoff enthaltende Stoffstrom als Seitenzulauf (35) zwischen Verstärkungsteil (31) und Abtriebsteil (33) zugeführt wird.

4. Verfahren gemäß einem der Ansprüche 1 bis 3, **dadurch gekennzeichnet, dass** der am Kopf entnommene und Chlorwasserstoff enthaltende Stoffstrom (9) nach dem Komprimieren einer Verstärkerkolonne (43) zugeführt wird und der am Sumpf (45) der Verstärkerkolonne (43) anfallende Strom (47) am Kopf in die Destillationskolonne (1) zurückgeführt wird.

5. Verfahren gemäß einem der Ansprüche 1 bis 4, **dadurch gekennzeichnet, dass** der kondensierte und Chlorwasserstoff enthaltende Stoffstrom in dem Wärmeübertrager (60) Wärme an den zu kondensierenden, Chlorwasserstoff enthaltenden Stoffstrom abgibt, bevor dieser komprimiert wird.

6. Verfahren gemäß einem der Ansprüche 1 bis 5, **dadurch gekennzeichnet, dass** der nicht kondensierte Teil des Chlorwasserstoff enthaltenden Stoffstroms in einem Wärmetauscher (65) von dem komprimierten zu kondensierenden Chlorwasserstoff enthaltenden Stoffstrom Wärme aufnimmt.

7. Verfahren gemäß einem der Ansprüche 1 bis 6, **dadurch gekennzeichnet, dass** der Destillationskolonne ein Gasstrom über einen Seitenabzug (53) entnommen wird, dieser in einem ersten Kühler (55) zumindest teilweise kondensiert wird, der flüssige Anteil in die Destillationskolonne (1) zurückgeführt wird und der gasförmige Teil einem zweiten Kühler (59) zugeführt wird, wobei der Gasstrom im zweiten Kühler (59) Wärme an den am Kopf entnommenen und Chlorwasserstoff enthaltenden Stoffstrom abgibt.

8. Verfahren gemäß Anspruch 7, **dadurch gekennzeichnet, dass** der am Kopf entnommene Stoffstrom (9) vor Zufuhr in den zweiten Kühler (59) in einem Wärmeübertrager (60) aufgeheizt wird.

9. Verfahren gemäß Anspruch 8, **dadurch gekennzeichnet, dass** im Wärmeübertrager (60) der am Kopf entnommene Stoffstrom (9) Wärme von dem am Kopf in die Destillationskolonne zurückgeführten kondensierten Stoffstrom aufnimmt.

## Claims

1. A method of separating a phosgene- and hydrogen chloride-comprising stream (5; 35), wherein said method comprises conveying the hydrogen chloride- and phosgene-comprising stream (5; 35) into a distillation column (1), withdrawing at the bottom of the distillation column (1) a phosgene-comprising stream (7) and withdrawing at the top of the distillation column (1) a hydrogen chloride-comprising stream (9), wherein at least a portion of the stream (9) withdrawn at the top is compressed and at least partially condensed and at least a portion of the liquid and compressed hydrogen chloride-comprising stream is decompressed and recycled into the top of the distillation column (1) as reflux.

2. The method according to claim 1, wherein the stream (9) is withdrawn at the top at a pressure in the range of from 1 to 10 bar and is compressed to a pressure in the range of from 5 to 25 bar before condensation.

3. The method according to either of claims 1 and 2, wherein the distillation column (1) comprises a rectifying section (31) and a stripping section (33) and the phosgene- and hydrogen chloride-comprising stream is supplied as side feed (35) between the rectifying section (31) and the stripping section (33) .

4. The method according to any of claims 1 to 3, wherein after compression the hydrogen chloride-comprising stream (9) withdrawn from the top is supplied to a rectifying column (43) and the stream (47) obtained at the bottom (45) of the rectifying column (43) is recycled into the top of the distillation column (1).

5. The method according to any of claims 1 to 4, wherein in the heat transferor (60) the condensed and hydrogen chloride-comprising stream gives off heat to the hydrogen chloride-comprising stream that is to be condensed, this giving-off of heat occurring before said stream is compressed.

6. The method according to any of claims 1 to 5, wherein in a heat exchanger (65) the uncondensed portion of the hydrogen chloride-comprising stream absorbs heat from the compressed and hydrogen chloride-comprising stream that is to be condensed.

7. The method according to any of claims 1 to 6, wherein a gas stream is withdrawn from the distillation column via a side takeoff (53), said stream is at least partially condensed in a first cooler (55), the liquid fraction is recycled into the distillation column (1) and the gaseous portion is supplied to a second cooler (59), wherein in the second cooler (59) the gas stream gives off heat to the hydrogen chloride-comprising stream withdrawn from the top.

8. The method according to claim 7, wherein the stream (9) withdrawn from the top is heated up in a heat transferor (60) before being fed into the second cooler (59).

9. The method according to claim 8, wherein in the heat transferor (60) the stream (9) withdrawn from the top absorbs heat from the condensed stream recycled into the top of the distillation column.

## Revendications

1. Procédé de séparation d'un courant de matière contenant du phosgène et du chlorure d'hydrogène (5 ; 35), le courant de matière contenant du chlorure d'hydrogène et du phosgène (5 ; 35) étant introduit dans une colonne de distillation (1), un courant contenant du phosgène (7) étant soutiré au fond de la colonne de distillation (1) et un courant de matière contenant du chlorure d'hydrogène (9) étant soutiré à la tête de la colonne de distillation (1), **caractérisé en ce qu'**au moins une partie du courant de matière (9) soutiré à la tête est comprimée et au moins partiellement condensée, et au moins une partie du courant de matière contenant du chlorure d'hydrogène liquide et comprimé est détendue, et recyclée en tant que reflux à la tête dans la colonne de distillation (1) .

2. Procédé selon la revendication 1, **caractérisé en ce que** le courant de matière (9) est soutiré à la tête à une pression dans la plage allant de 1 à 10 bar et comprimé avant la condensation à une pression dans la plage allant de 5 à 25 bar.

3. Procédé selon la revendication 1 ou 2, **caractérisé en ce que** la colonne de distillation (1) comprend une zone d'enrichissement (31) et une zone de rectification (33), et le courant de matière contenant du phosgène et du chlorure d'hydrogène est introduit en tant qu'entrée latérale (35) entre la zone d'enrichissement (31) et la zone de rectification (33).

4. Procédé selon l'une quelconque des revendications 1 à 3, **caractérisé en ce que** le courant de matière (9) soutiré à la tête et contenant du chlorure d'hydrogène est introduit après la compression dans une colonne d'enrichissement (43), et le courant (47) formé au fond (45) de la colonne d'enrichissement (43) est recyclé à la tête dans la colonne de distillation (1).

5. Procédé selon l'une quelconque des revendications 1 à 4, **caractérisé en ce que** le courant de matière condensé et contenant du chlorure d'hydrogène émet de la chaleur vers le courant de matière contenant du chlorure d'hydrogène à condenser dans l'échangeur de chaleur (60) avant la compression de celui-ci.

6. Procédé selon l'une quelconque des revendications 1 à 5, **caractérisé en ce que** la partie non condensée du courant de matière contenant du chlorure d'hydrogène absorbe de la chaleur à partir du courant de matière contenant du chlorure d'hydrogène comprimé à condenser dans un échangeur de chaleur (65).

7. Procédé selon l'une quelconque des revendications 1 à 6, **caractérisé en ce qu'**un courant gazeux est soutiré de la colonne de distillation par une sortie latérale (53), celui-ci est au moins partiellement condensé dans un premier refroidisseur (55), la partie liquide est recyclée dans la colonne de distillation (1) et la partie gazeuse est introduite dans un deuxième refroidisseur (59), le courant gazeux émettant de la chaleur vers le courant de matière soutiré à la tête et contenant du chlorure d'hydrogène dans le deuxième refroidisseur (59).

8. Procédé selon la revendication 7, **caractérisé en ce que** le courant de matière (9) soutiré à la tête est chauffé dans un échangeur de chaleur (60) avant l'introduction dans le deuxième refroidisseur (59).

9. Procédé selon la revendication 8, **caractérisé en ce que** le courant de matière (9) soutiré à la tête absorbe de la chaleur à partir du courant de matière condensé recyclé à la tête dans la colonne de distillation dans l'échangeur de chaleur (60).
